# EUROPEAN PATENT APPLICATION

(11) **EP 1 603 069 A1**
(43) Date of publication of application: **07.12.2005**
(21) Application number: 04253344.8
(22) Date of filing: 04.06.2004
(51) Int. Cl.: G06F 19/00

(54) **Information service system based on genetic character**

(71) Applicant: Kurita, Hiromasa, Chiba-shi, Chiba (JP); Sera, Hiroko, Tokyo (JP); Mizusaako, Kazuko, Akishima-shi, Tokyo (JP); Sera, Koji, Tokyo (JP); Kurita, Masumi, Chiba-shi, Chiba (JP); Sera, Naoto, Tokyo (JP)
(72) Inventor: Kurita, Hiromasa, Chiba-shi, Chiba (JP); Sera, Hiroko, Tokyo (JP); Mizusaako, Kazuko, Akishima-shi, Tokyo (JP); Sera, Koji, Tokyo (JP); Kurita, Masumi, Chiba-shi, Chiba (JP); Sera, Naoto, Tokyo (JP)
(74) Representative: Finnie, Peter John

(57) **Abstract**

To provide genetic information, etc. concerning user constitution to the user, apply the static technique to the information, carry out detailed analyses, and improve the living including eating habit, etc.

The user terminal, and the inspection section, and the server are connected to the user terminal via networks, and when the inspection section receives an inspection inquiry from the user terminal, the inspection section transmits inquiry data to the user terminal, the subject enters replies to the transmitted inquiry data into the user terminal and transmits the entered data from the user terminal to the inspection section, and at the same time, presents samples that serve as inspection media of genetic characters, and the inspection section inspects the genetic characters of the subject in accordance with the transmitted inquiry data and samples presented, and transmits the inspection results to the user terminal as electronic information or to the place specified by the subject in writing.

## Description

### Field of the Invention

The present invention relates to an information service system based on genetic characters comprising an organ for grasping human genome information of the user proper by inspections, etc., presenting disease contracting possibility including genetic characters to the user, judging whether part of the information is a new genetic character using statistics, etc., and inspecting the method for improving the user living and genetic information including user and human genome as well as a brain for confirming and theorizing the presence of genetic information by carrying out academic analysis, , and providing a method for better living to the user.

### Background to the invention

In recent years, the human genome analysis is carried out worldwide, and nearly 70% analysis has been already completed, and there partly exists the information that all the analysis has been completed. Based on the analysis of the human genome, people's attention has been drawn to inherited diseases which have been told heretofore, such as diabetes, heart disease, stroke, allergy, atopy, chemical substance anaphylaxis, and others, or the phenomena in which a certain genetic character suddenly awakes or is actualized when a user reaches a certain age, and there exists the information that the number of users who want to grasp these genetic characteristics in advance and incorporate them into their every-day life exceeds hundreds of thousands. For example, it may be elucidated that when a parent contracts an organ cancer such as lung cancer, etc., the child has a possibility to contract some kind of organ cancers at the time when his/her parent came down with a cancer or even if the child contracts the same organ cancer, the disease is developed earlier than the onset of the disease of his/her parent.

On the other hand, it has been pointed out that since the human genome information contains the individual information of that person, disclosing the information to any third party casually may violate the individual privacy, resulting in disadvantage to that person, for example, the person may be treated disadvantageously than others in signing the policy, and consideration must be given to careful handling.

Under these circumstances, there is a possibility that it might be requested to find out a social harmony so that with the privacy of each individual protected, the individual genetic information could be utilized for academic knowledge or the healthy and civilized mode of living could be enhanced by individually acquiring the knowledge on genetic characters, and as a result, it could contribute to improvement of the public welfare, etc. of the whole nation. For example, it is pointed out that when a parent has an experience of organ cancer, etc. as described above, the genetic information may be applied to the case of the child, for example, the case in which the child may die from the organ cancer, and this may be of help to promote the welfare as described above.

Under these circumstances, first of all, any gene research agency has not yet been established, which could give any answer to the right of a general user to know the inherited characteristics which the user has. In addition, there are a bunch of very real problems kept even undiscussed, for example, how and when should be predicted and coped with a certain kind of inherited disease whose development could be foreseen, how the liaison should be established with specialist medical institutions, and no infrastructure has been built up as to how such organization should be created for such purposes.

When these medical institutions are formed, it is requested to provide the information on the above-mentioned improvement to the people who have a unique constitution, which is difficult to be included in the diagnosis or treatment related to inherited medical histories (including the possibility of manifestation in the future), such as constitution easy to get fat, constitution to become taller than ordinary people, though it does not go to the gigantism, constitution to easily develop diabetes, with a hope to prevent these people, who have the characteristics possibly called backup candidates of diseases, from contributing to contraction of diseases by improving the environmental characteristics such as dietary life, etc. By providing the information to such people, it is requested to prevent diseases such as lung cancer.

It is an object of the present invention to provide the genetic information, etc. concerning the constitution of a user who has been scientifically proven to have so-called backup candidate characteristics of diseases, that the user hereditarily possesses a constitution likely to get fat or contract diabetes, or with high possibility to contract lung cancer and other diseases when living environment factors including dietary life environment, such as tobacco, etc. are given.

It is another object of the present invention to build up a flexible system that can elucidate diseases which are not thought to be genetic characters or improve the life including dietary life, etc. of the user by analyzing the genetic character information including human genome information from the user by the use of statistical techniques, etc. or by giving doctor inquiries.

### Summary of the Invention

In order to solve the above-mentioned problems, an information service system based on the genetic characters related to the present invention comprises one or more user terminals possessed by the subjects, an inspection section for inspecting the genetic characters, a server for operating the system, all of which are connected via networks, and when the inspection section receives the application for inspection from the user terminal, the inspection section transmits the doctor inquiry data to the user terminal, and the subject enters the replies to the transmitted doctor inquiry data into the user terminal, and transmits the entered data from the user terminal to the inspection section and at the same time, provides a specimen which serves as genetic character inspection medium to the inspection section, and the inspection section examines the genetic characters of the subject in accordance with the transmitted inquiry data and samples presented, transmits the inspection results to the user terminal as the electronic information, or gives a notice in writing to the designated place of the subject.

In such event, the inspection results include advice to the subject concerning living environment and diet

In addition, the information service system has a subject database for recording inspection results by the inspection section in accord with subjects.

In addition, the information service system has a brain section for judging whether or not the extracted inspection results are the information which may be disclosed or not when the inspection results recorded in the subject database is extracted in compliance with the desired extraction conditions via the inspection section.

In addition, the information service system has at least one or more industrial terminals connected, wherein the brain section transmits the inspection results of genetic characters of the user from the inspection section to the industrial terminal after the inspection by the inspection section is completed.

Furthermore, judgments to protect user privacy are included in the judgments of the inspection results by the brain section.

### Brief Description of the Drawings

Examples of the present invention will now be described in detail with reference to the accompanying drawings in which:
Fig. 1 is a diagram that indicates one example of genetic characters inspection system configuration according to the present invention;
Fig. 2 is a diagram indicating an example that stipulates inquiries (doctors questions) to the user;
Fig. 3 is a diagram showing one example of format for notifying the inspection results transmitted to the user;
Fig. 4 is a diagram indicating another example of the configuration of genetic characters inspection system according to the present invention; and
Fig. 5 is a graph that plots the relationship between names of foreseeable diseases attributed to genetic characters (abscissa) and various industries.

### Detailed Description

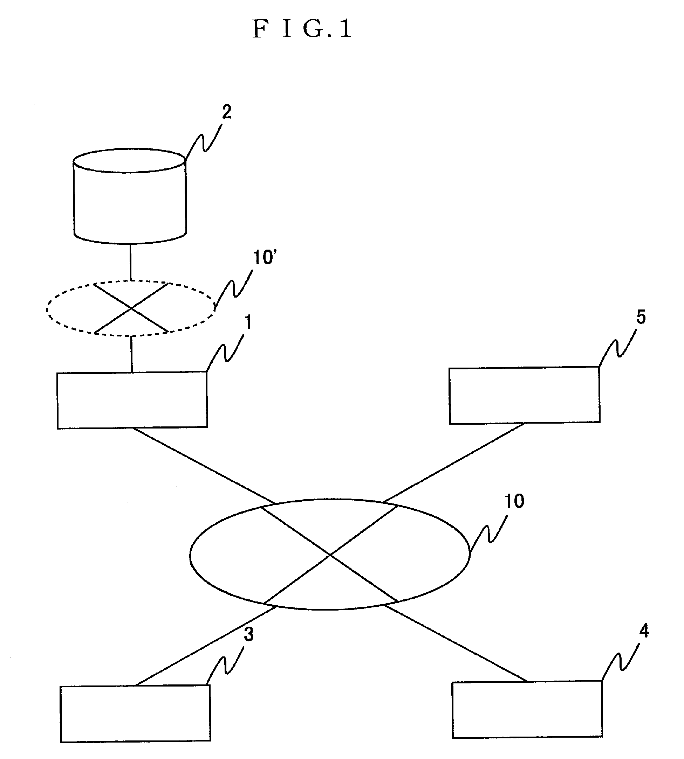

As shown in Fig. 1, the inspection system of genetic characters related to the present invention comprises an inspection section 1 for inspecting user hereditary constitution, a genetic information storage section 2 for storing the inspected genetic information, a storage section for receiving samples sent and entering and storing the user data for inspecting the genetic characters, and a user terminal 4. These are connected one another, for example, via network 10 or via WAN and LAN. Furthermore, through the network, terminals 5 of pharmaceutical companies, etc. may be connected to the present system, and a system server 3 for administering the whole system of this kind may be connected to the network.

### Inspection Section 1

Users bring by or send something that enables the analysis of at least part of their gene by blood, urine, etc. as a sample for their own gene analysis directly or by transmission methods such as mail, etc. to the inspection section 1. Preferably, as a sample collection method, a sampling method accompanied by no pain may be adopted when samples are collected from users. For example, as this kind of sample, user nail, saliva, urine, and any other bodily wastes, or body hair including hair on the head may be mentioned.

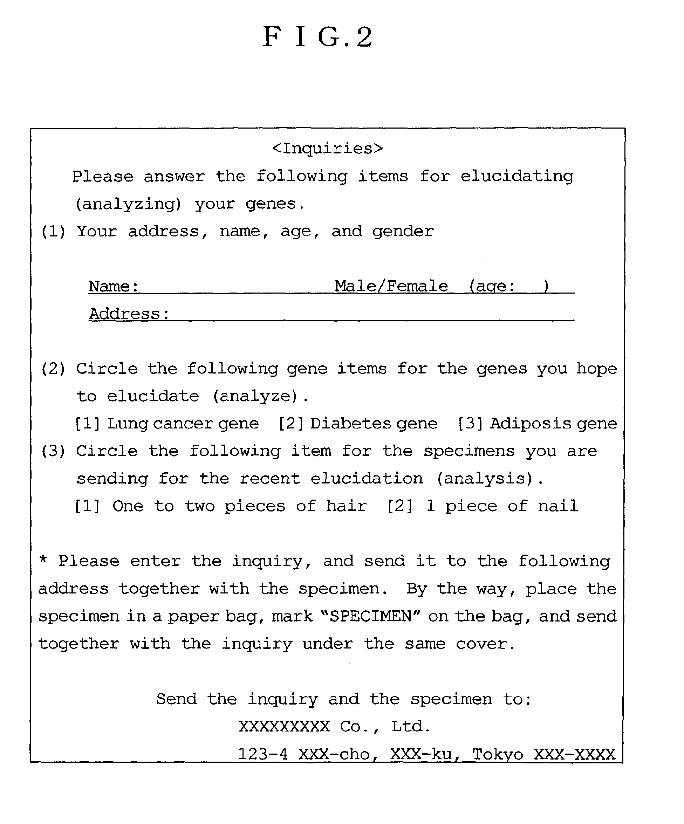

The inspection section 1 (inspection institution) confirms whether or not the necessary information from the user is attached before or after acquiring this kind of sample, and undertakes inspection in accordance with the inspection items desired by the user. The gene characters can be inspected by adopting any known methods. By the way, the inspection section 1 receives orders of inspection from the user via the user terminal 4 or directly from the user to the inspection section 1. The inspection section 1 directly asks the user about his/her condition or transmits the inquiry data (interview sheet) to the user terminal as shown in Fig. 2 through network after receiving the order.

After receiving replies to the doctor's questions directly from the user or replies to the inquiry data from the user terminal through network, the inspection section 1 makes sure the format of the doctor's questions has the specified content duly entered. If it is checked by this entry check that the content of the specified items is all entered satisfactorily, the inspection section 1 at least begins the analysis (diagnosis) of inspection items which the user desires. Depending on the circumstances, the inspection section 1 may increase and analyze the inspection items for determining genetic characters as required in addition to the inspection items desired by the user.

### Subject Database

The results (including gene information) of inspecting or elucidating this kind of user genetic characters may be saved in the subject DB (data storage) 2 which the inspection section (inspection institution) 1 possesses and stored in memory, but the subject DB 2 may be recorded in the external data recording section.

Then, the inspection section 1 judges whether or not the user has the genetic character which the user hopes to learn using the inspection results of user genetic characters obtained, and if the user possesses the genetic character, the inspection section 1 prepares advice items, for example, in daily life or daily dietary life, for the user.

The document prepared is converted, for example, into an electronic mail format, and sent to the user, preferably, via network. Because the user who possesses the user terminal 4 extracts points that must be taken care of in his/her daily dietary life and daily life from the document transmitted, and leads snugly his/her real life while referring to the advice, etc. based on his/her own living philosophy, thinking, etc., the user could accept precisely the advice, even for the intake time and intake amount of drugs which the user has taken and ingested without particular awareness, and this would contribute to improvement of user's physical condition or improve his/her physical shape in the future, even though the user does not feel well at the present moment.

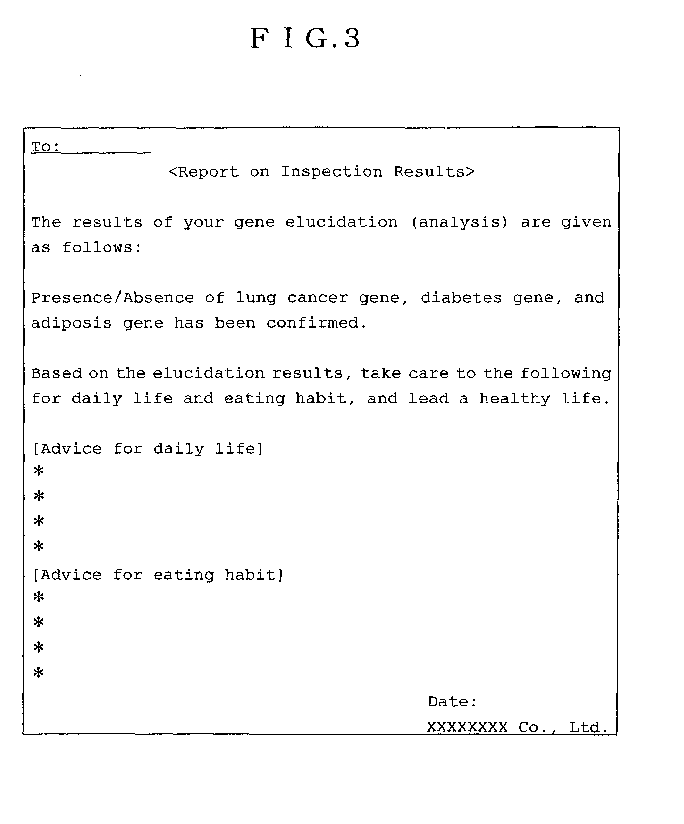

In addition, the user can receive carefully thought advice, such as staggering the commuting time zone, meal-taking time or intake, changing the meal quality to high protein or high mineral (high in fiber), and so forth. Fig. 3 indicates one example of the inspection report format by which this kind of inspection results is notified to the user. In the format shown in this Fig. 3, the presence of lung cancer gene, etc., items advised with respect to the daily life, and items advised for diet meals are indicated. This format shows only one example to be transmitted (including forwarding by mail) to the user, and the setting may be varied as required, for example, depending on the circumstances, adding the genetic characters such as high blood pressure, etc. or giving advice such as special notations to genetic characters of user's spouse, etc.

In the present system, when an electronic mail including this kind of advice is transmitted or in other occasions, billing processing may be carried out or billing processing may be executed suitably when the user applies for the inspection.

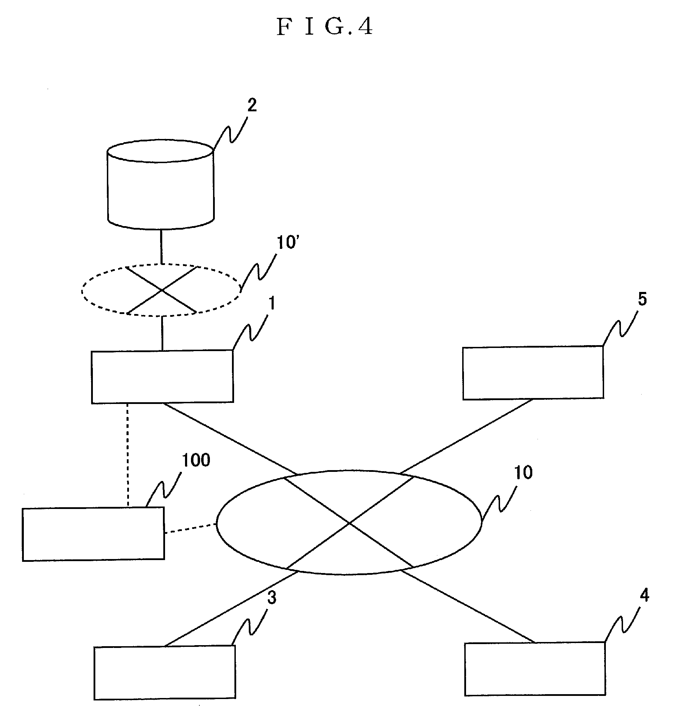

Positively making the best of the genetic character information of the subject who possesses this kind of user terminal 4, the system may be built as an information service system which has a brain section 100 as depicted in Fig. 4. This brain section 100 can carry out analyses from the scientific viewpoint to analytically investigate the genetic characters or extract cases by doctor's questions, etc. in addition to the inspection items of the user genetic characters and reflect the results to the advice to the user, or can follow cases of the user thereafter if a new gene analysis method is adopted or the genetic character is assumed to be related to the cases which have not yet been known before.

In addition, the brain section 100 enables the appropriate use of the information on genetic characters from the medical viewpoint. Consequently, brain section 100 judges in conformity to the following items as to whether or not the information including genetic characteristics should be fumished in various industries, such as apparel industry, food industry, real estate industry, pharmaceutical industry (for example, including senior citizens), health care industry (for example, including senior citizens), cosmetics industry (for example, including senior citizens), life insurance industry, manpower supply industry, tobacco industry, airline industry, transport industry, marriage-mediation business industry, and forestry, livestock industry, educational industry, etc.

### Judgment Items

1. Individual information (or information of an unidentified person: protection of privacy)?
2. Information with strong personal colors or information with strong statistical colors?
3. In the case of Paragraph 1 or 2 above and in the case of personal information or information with strong personal colors (these pieces of information are called the "personal information"), may the individual privacy be highly likely to be protected?
4. Is the disclosure one-sided to a specific company of a certain industry?
5. Is the disclosure made to the payable industry?
6. New business?
7. Is there any disclosure which intends to use the information including genetic characteristics?
In accordance with these judgment items, the brain section 100 decides the kind of votes, such as decision by majority, more than 2/3, or unanimous vote, etc.; then, judges whether the information should be disclosed or not, and in the case of disclosure, the brain section 100 decides the scope of disclosure, and records the judgment results in the storage means inside the brain section 100. This record is preferably write-only and overwrite-inhibited. In addition, it is set that all the access records are, in principle, recorded. Furthermore, specific content of the judgment results (kind of vote, specific name of assentients, content of assenting opinions, name of dissidents, and content of dissenting opinions, and all other content of the minute) are, in principle, recorded.

In the brain section 100, when the disclosure of information containing the genetic characteristics is decided in this way, it is desirable to disclose the brief content of genetic characteristics disclosed, content including brief comments of the progress of the minute, and brief content of disclosing industry, etc. as a summary, for example, www (world wide web) via Internet. However, in a certain case, this kind of disclosure may be carried out to a specific company only.

For example, in the event that a clinical trial of a specific drug for a certain cancer is requested by one company in the health-care industry, even when it is foreseen that taking the specific drug together with drug A for diabetes causes a fatal disease to be developed, the brain section 100 can prevent the specific patients from being administered with this specific drug or searches cancer patients by following users recorded in the subject DB 2 and carries out the clinical trial, etc. Such case is rather a case to notify the subjects as specific persons to the company or institutions including hospitals, etc. It is also possible to take in the functions of the brain section 100 into the system server 3 for processing.

In addition, when any disease case which has never been foreseen in a certain medicinal product by a specific prescription, the subject DB, etc. are searched, and if any user exists as a subject, the relation with the genetic characters described above may be investigated and the countermeasures may be worked out. Or, for the patient who has the similar genetic characteristics after any disease case occurs, a room for investigating the administration is provided. In such case, there is a possibility to carry out the investigation using a statistical analysis (diagnosis) without specifying the patient or user name.

Furthermore, there occurs a possibility that the brain section 100, etc. which takes initiative to analyze and search the subject DB 2 clarifies and foresees the effects of composite prescription of drug A and drug B, relevancy between the food and chemical substance anaphylaxis, environmental factors, etc. and genetic characteristics, which have been overlooked before. For example, users who have drug A administered (setA) are extracted, users who have drug B administered (set B) are extracted, the condition, etc. of users (setA Λ B) who are receiving the composite prescription of product set of drug A and drug B are compared with, for example, that of set A-A Λ B, set B-A Λ B, and other users, and the relevancy can be judged with statistical priority taken into account.

This kind of cases, etc. may be predicted by analysis (diagnosis) processing by the use of statistical technique without specifying subjects.

In addition, by using the system of the present invention, the system can be utilized for the future pharmaceutical business, etc. by expertly adding the genetic character information of a new business.

According to the system of the present invention, for example, it is assumed that patients who hope to have the high-blood pressure contracting gene elucidated exist in the number of about one million as a reserve for high-blood pressure disease candidates. Similarly, patients, etc. who desire to have the heart disease contracting gene clarified are predicted to exist in the number of about 500,000, whereas patients who desire to have the stroke (cerebral hemorrhage) contacting gene exist in the number of about 500,000, similarly, and patients, etc. who desire to have the allergic disease contract gene elucidated is assumed to exist about 700,000. Therefore, by analyzing genes of the users who are like reserves of these diseases, subject DB 2 may be prepared and may be utilized as the database which is useful for new business.

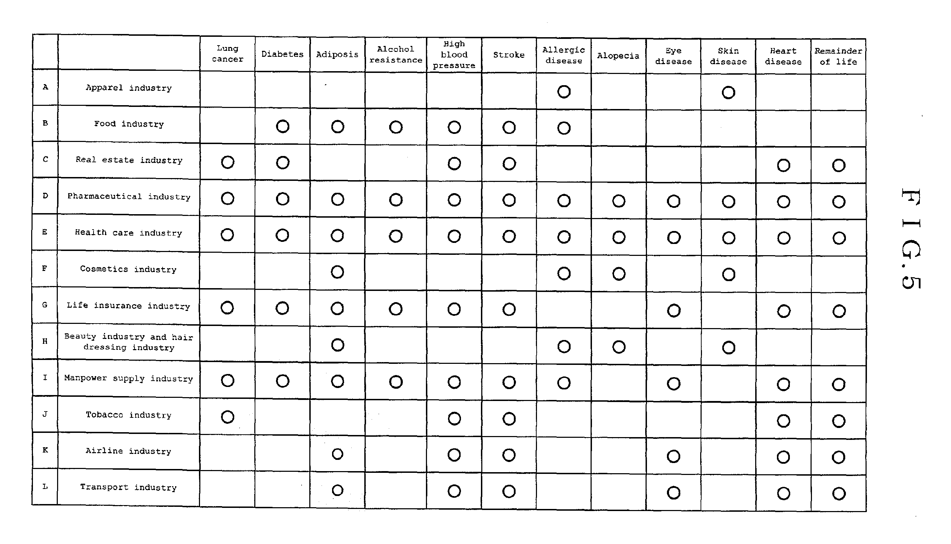

Fig. 5 depicts one example of new business which predicts the relationship between these genetic characters and related industries.

As shown in Fig. 5, the present invention includes compilation of a database of the results obtained from the analysis of genetic characters including user gene analysis as well as creation of businesses of various related industries.

For users, the present invention also notifies the results of the elucidation (analysis) of genetic characters of the user proper and gives advice to reduce the possibility of hereditary diseases or contraction of diseases by learning the results; sells dietary supplements or health supplements to users; supports the users in their living phase (including spiritual phase) by allowing the users to enjoy the health control method including new medical method; helps various industries develop fixtures, foods and beverages, food additives, cosmetics, or clothing that could suppress, for example, atopic dermatitis as described above; and for users who have Alzheimer's genetic characters, the present invention supports their living phase (including spiritual phase) as described above, supports them to develop fixtures, foods and beverages, food additives, and clothing; backs up for disseminating fixtures to prevent atopy, and supports various industries including educational support, etc. Consulting, etc. including these supports (including management support) are also included in the present invention.

As clear from the above description, according to the present invention, it is possible to embody a system that can prepare a subject DB and provide the information useful for new businesses by carrying out gene analyses of users who are assumed to be disease contracting reserves of a high probability of contracting the diseases in the future, which are caused by various genetic characters.

In particular, if a configuration is adopted with a brain section arranged to the information service system based on the genetic characters of the present invention as shown in Fig. 4, it is possible to create an organization utilizing a new system or participate in a new business because it is possible to predict the time when the hereditary disease would be manifested and to take necessary actions or expert medical institutions or specialist knowledge can be utilized since the relevancy between the gene and contraction ratio can be investigated in detail.

For example, based on the inspection results, advice may be given such as considerations, etc. of genetic characters of the spouse, etc., but based on this kind of information, in the marriage-mediation business, the inspection results may be notified to spouse candidates with hereditary elements added, thereby contributing to the mediation business.

## Claims

1. An information service system based on genetic characters comprising:
a user terminal possessed by one or more subjects;
an inspection section for inspecting genetic characters; and
a server for operating the system, all of which are connected via networks,
wherein the inspection section transmits the inquiry data to the user terminal when the section receives an inspection application from the user terminal, the subject enters the replies to the inquiry data transmitted to the user terminal and transmits the entered data from the user terminal to the inspection section as well as provides samples which serve as inspection media of the genetic characters to the inspection section,
wherein the inspection section inspects genetic characters of the subject based on the inquiry data transmitted and the specimens provided, transmits the inspection results on the genetic characters to the user terminal as electronic information or gives notice to the place specified by the subject in writing.

2. The information service system based on genetic characters according to claim 1,
wherein the inspection results include advice concerning living environment and a diet of the subject.

3. The information service system based on genetic characters according to claim 1 or claim 2, wherein the information service system has a subject database for recording inspection results by the inspection section in accord with subjects.

4. The information service system based on genetic characters according to claim 3, further comprising:
a brain section for judging whether or not the extracted inspection results are the information which may be disclosed or not when the inspection results recorded in the subject database is extracted in compliance with the desired extraction conditions via the inspection section.

5. The information service system based on genetic characters according to claim 4, further comprising:
at least one or more industrial terminals,
wherein the brain section transmits the inspection results of genetic characters of the user from the inspection section to the industrial terminal after the inspection by the inspection section is completed.

6. The information service system based on genetic characters according to claim 4, wherein judgments to protect user privacy are included in the judgments of the inspection results by the brain section.
